# EUROPEAN PATENT SPECIFICATION

(11) **EP 1 763 343 B1**
(45) Date of publication and mention of the grant of the patent: **30.12.2009**
(21) Application number: 05758830.3
(22) Date of filing: 17.06.2005
(51) Int. Cl.: A61K 31/19, A61P 9/10, A61P 9/02, A61P 17/06, A61P 17/00, A61P 43/00

(54) **CALCIUM TRIFLUOROACETATE FOR PREPARING ANTIANGIOGENETIC MEDICAMENTS**
CALCIUMTRIFLUOROACETAT ZUR BEREITUNG ANTIANGIOGENETISCHER ARZNEIMITTEL
TRIFLUROACETATE DE CALCIUM POUR LA PREPARATION DE MEDICAMENTS A EFFET ANTIANGIOGENETIQUE

(30) Priority: 24.06.2004 IT MI20041279
(43) Date of publication of application: 21.03.2007
(73) Proprietor: Eureon AG, 79104 Freiburg (DE)
(72) Inventor: GOBBI, Rosa, I-22073 Fino Mornasco (CO) (IT)
(74) Representative: Bianchetti, Giuseppe
(86) International application number: PCT/EP2005/006533
(87) International publication number: WO 2006/000339

(56) References cited:
- EP-B1- 1 343 779
- WO-A-01/68070
- WO-A-03/006031
- WO-A-2005/097084

## Description

The present invention relates to the use of calcium trifluoroacetate for the preparation of medicaments with antiangiogenetic effect.

### TECHNOLOGICAL BACKGROUND

Angiogenesis is a process leading to formation of new vessels, connected with blood circulation, through activation of vascular endothelial cells which are part of pre-existing capillaries and venules. In response to angiogenetic stimuli, the endothelial cells migrate into the perivascular space, where they proliferate and form the novel vessels.

In the adult, endothelial cells show very slow turnover, and angiogenesis is restricted to physiological conditions such as wound reparation and cyclic processes of the female reproductive system (ovulation, menstruation, implant and pregnancy). Physiological angiogenesis is strictly controlled, activated for short periods, only as far as tissue metabolic requests are concerned, then promptly inhibited.

Angiogenesis is also the "common denominator" of a number of pathological conditions in humans. It is in fact involved in the vascularization of the atherosclerotic plaque, promoting its instability and formation of thrombi, in the tissue damage deriving from rheumatoid arthritis and psoriasis, in the development of diabetic retinopathy, as well as in the development of solid tumors. In particular, it is established that a tumor cannot grow beyond a few millimetres in the absence of vascularization. Moreover, tumour angiogenesis promotes invasivity and metastatic diffusion of tumours.

EP 1 423 131 discloses that calcium trifluoroacetate and related calcium salts inhibit tumor growth in animal experimental models and exert *in vitro* cytotoxic activity on solid tumor cells.

### DISCLOSURE OF THE INVENTION

It has now been found that calcium trifluoroacetate and related salts disclosed in EP 1 423 131, in addition to cytotoxic and antitumor activity on solid tumors, have surprisingly marked antiangiogenetic activity.

The effect of calcium trifluoroacetate in angiogenesis was evidenced in a murine model of angiogenesis induced by a subcutaneous implant of Matrigel. Matrigel is liquid at 4°C and solid at 37°C, so that a pro-angiogenetic stimulus can be mixed with Matrigel and inoculated. In this protocol, the selected angiogenetic factor was endothelial growth factor (VEGF), added to the Matrigel together with 16 U/ml heparin, as described in literature (Bussolati B, Altered angiogenesis and survival in human tumor-derived endothelial cells. FASEB J. 2003; Bussolati B, Vascular endothelial growth factor receptor-1 modulates vascular endothelial growth factor-mediated angiogenesis via nitric oxide. Am J Pathol. 2001).

Calcium trifluoroacetate was administered intraperitoneally 30 minutes before Matrigel, then on alternate days (day 2, 4,6), at a dose of 2 or 4 µg/mouse (0.2 mg/Kg). After 7 days, the animals were killed and the Matrigel plugs were removed for histological examination. Angiogenesis was evaluated by computer assisted morphometric analysis of the percentage of area covered by vessels.

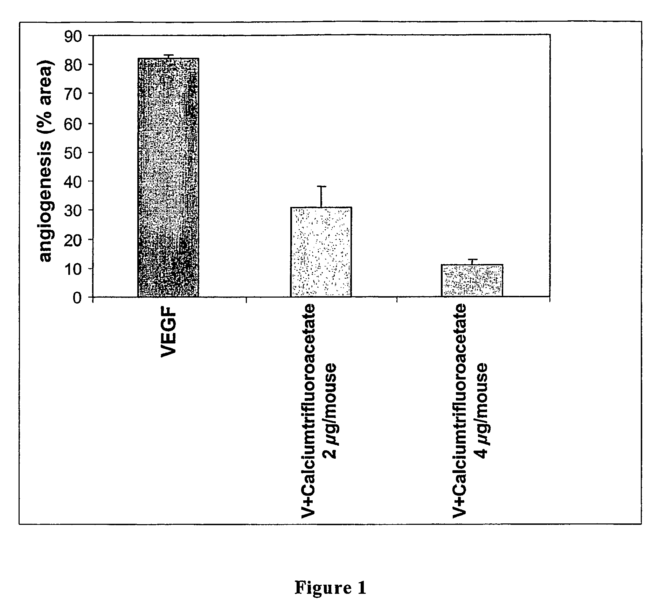

Figure 1 shows the reduction of angiogenesis in mice treated with VEGF + calcium trifluoroacetate on alternate days at a dosage of 2 µg/mouse (n=4) and of 4 µg/mouse (n=8) compared with VEGF alone (n=8). P<0.001.

A marked angiogenesis was observed in the Matrigel plugs containing VEGF (40 ng/ml), as expected. The effect of VEGF was dramatically reduced in mice treated with calcium trifluoroacetate. This strong antiangiogenic effect could already be observed macroscopically and confirmed histologically. Morphometric analysis showed a reduction in angiogenesis above 40% with a dosage of 2 µg/mouse of calcium trifluoroacetate, and of 80% with a dosage of 4 µg/mouse.

Calcium trifluoroacetate has evident inhibitory action on endothelium proliferation. Moreover, calcium trifluoroacetate is able to block, at least partly, the effect of the usual endothelial growth factors on calcium entrance into the single endothelial cells.

These data suggest that calcium trifluoroacetate can inhibit the response of endothelial cells to stimulation by growth factors, likely acting on the message transmission through the calcium channels.

Calcium trifluoroacetate can therefore be successfully used, in the form of suitable pharmaceutical compositions, for the treatment of pathologies in which the inhibition of angiogenesis is necessary or appropriate, which are atherosclerotic plaque, rheumatoid arthritis, psoriasis, diabetic retinopathy, rosacea, cheloids

For the envisaged therapeutical uses, calcium trifluoroacetate will be administered through the oral, topical, transdermal or parenteral (subcutaneous, intramuscular or intravenous) routes at dosages similar to those disclosed in EP 1 423 131, for example ranging form 20 to 100 mg/kg for the oral and parenteral routes, or at concentrations ranging from 2.5⁻ to 10% for the topical formulations.

Calcium trifluoroacetate may optionally be combined with other active ingredients having complementary or anyway useful activity.

## Claims

1. The use of calcium trifluoroacetate for the preparation of medicaments for the treatment of atherosclerotic plaque, rheumatoid arthritis, psoriasis, diabetic retinopathy, rosacea, cheloids.

## Patentansprüche

1. Verwendung von Kalziumtrifluoracetat zur Herstellung von Medikamenten zur Behandlung von atherosklerotischer Plaque, rheumatoider Arthritis, Psoriasis, diabetischen Retinopathie, Rosacea, Cheloiden.

## Revendications

1. Utilisation de trifluoroacétate de calcium pour la préparation de médicaments pour le traitement de la plaque athéroscléreuse, de la polyarthrite rhumatoïde, du psoriasis, de la rétinopathie diabétique, de la rosacée, des chéloïdes.
